# EUROPEAN PATENT APPLICATION

(11) **EP 3 590 883 A1**
(43) Date of publication of application: **08.01.2020**
(21) Application number: 19179859.4
(22) Date of filing: 13.06.2019
(51) Int. Cl.: B67B 3/00, F26B 21/00

(54) **HOT FLUID SUPPLY CONDUIT CONFIGURED FOR DRYING OF ARTICLES AND STERILIZATION UNIT COMPRISING SAID CONDUIT**

(30) Priority: 21.06.2018 IT 201800006553
(71) Applicant: Sidel Participations, 76930 Octeville-sur-Mer (FR)
(72) Inventor: FAVA, Fabrizio, 43126 Parma (IT); PERRONE, Giovanni, 43126 Parma (IT)
(74) Representative: Sidel Group

(57) **Abstract**

The invention refers to a hot fluid supply conduit (1) configured to dry articles (2), comprising a first tubular wall (10) extending along an axis (A) and internally defining a first flow channel (11) for the hot fluid having a first axial end portion (12) and a second axial end portion (13) arranged on axially opposite sides of the first channel (11); the first tubular wall (10) comprises a back wall (14) arranged at the second end portion (13) and configured to axially close the first channel (11); the latter is configured to receive the hot fluid and to feed it to the articles (2) through a plurality of outlet openings (15); the conduit (1) further comprises a second tubular wall (16), arranged at least partially inside the first tubular wall (10) and, defining a second flow channel (17) for the hot fluid, having a through opening (18) adapted to be fluidically connected to the outlet openings (15), facing the back wall (14) and arranged at a distance, other than zero, from the same; the second channel (17) is configured to convey the hot fluid towards the second end portion (13); the first tubular wall (10) and the second tubular wall (16) radially delimit, between one another, an annular region (20) configured to convey hot fluid from the second (13) to the first (12) end portion.

## Description

### TECHNICAL FIELD

The present invention relates to a hot fluid supply conduit that supplies, for example, hot air, configured to dry articles, in particular caps for containers such as bottles or the like, adapted to be filled with a pourable product, preferably a pourable food product.

The present invention furthermore relates to an article sterilization unit, in particular caps for containers such as bottles or the like, adapted to be filled with a pourable product, preferably a pourable food product.

### BACKGROUND ART

It is known that containers can be filled with different types of pourable food products, such as carbonated drinks, fruit juices, water, milk, etc.; the containers can be, for example, bottles having various shapes and sizes and made of different materials such as glass, plastic, aluminium etc.

The sterilization of such containers is of fundamental importance in order to guarantee the pre-set duration of the food products, and consequently, the safety of the consumers.

In general, the containers have respective inlet/outlet openings, through which the containers themselves are sterilized and subsequently filled, with the desired pourable product, inside a filling machine.

Once the filling operation has ended, the inlet/outlet openings are sealed by means of application and fixing of respective caps.

The caps can be of various types according to the various needs, for example screw caps, "sport" caps etc., and are generally made of plastic or metallic material.

In particular, said caps must also be subjected to a sterilization treatment, carried out inside a suitable sterilization unit and, subsequently, are fed to a capping device, sometimes integrated in the filling machine.

The known sterilization units essentially include:
- a conveying structure that typically comprises a plurality of guide bars arranged so as to delimit a guide channel configured to receive the caps at an input station and guide the caps towards an output station; and
- a treatment chamber, crossed by the conveying structure and within which the sterilization takes place.

In detail, the guide bars define a track within which the caps slide, from the input station to the output station, simultaneously crossing the treatment chamber.

In this regard, the sterilization units of the above-described type typically comprise an injection pipe arranged inside the treatment chamber and adapted to inject a sterilizing agent therein, for example heated and vaporized hydrogen peroxide (VHP).

In this manner, a mist of sterilizing agent is formed through which the caps pass.

Typically, the sterilization units further comprise a drying device, generally a drying conduit positioned below a portion of the conveying structure, arranged outside of the treatment chamber and configured to supply a hot air flow towards said portion of the conveying structure, so as to touch the individual caps that slide therein.

In this way, the caps are dried of any possible residues of sterilizing agent.

Once the sterilization and drying process is completed, the caps are subsequently fed through the output station to the capping device, which is configured to apply each individual cap to a respective container.

Generally, the drying conduit comprises a tubular surface extending along an axis and internally defining a hot air flow supply channel.

In detail, the channel has an axial inlet opening configured to receive the hot air flow, and a plurality of outlet holes, arranged along a longitudinal band of the tubular surface, parallel to the axis, and configured to supply the hot air flow to the portion of the conveying structure above, and therefore towards the caps that slide therein.

With the aim of achieving a complete sterilization of the caps, the drying conduit must also be subjected to an appropriate sterilization.

In particular, it is known to carry out the sterilization of the drying conduit by making hot air flow therein, at a given sterilization temperature for a given period of time: these sterilization parameters are predetermined in the filling recipe concerning the containers to be filled.

The applicant has observed that in some applications in which a relatively extended drying conduit is necessary, it is difficult to obtain a substantially constant temperature profile along the entire conduit. In particular, the temperature tends to drop towards the final end of the supply channel.

The solution currently being used to deal with this problem consists in increasing the sterilization temperature; however, this practice results in an increase of the production costs.

Another solution used consists in increasing the time period during which the drying conduit is subjected to the sterilization temperature; however, this practice requires a change of the pre-set filling recipe and therefore an increase of the production costs.

A further possibility consists in sterilizing the drying conduit by injecting, into the supply channel, sterilizing chemical agents, which subsequently must be completely removed from the conduit.

### DISCLOSURE OF INVENTION

It is therefore an object of the present invention to provide a supply conduit, which which is designed to overcome at least one of the above-mentioned drawbacks in a straightforward and low-cost manner.

This object is achieved by a supply conduit as claimed in claim 1.

It is a further object of the present invention to provide a sterilization unit, which is designed to overcome at least one of the above-mentioned drawbacks in a straightforward and low-cost manner.

This object is achieved by a sterilization unit as claimed in claim 10.

### BRIEF DESCRIPTION OF THE DRAWINGS

For a better understanding of the present invention, a preferred but non-limiting embodiment will be described in the following, purely by way of example and with the aid of the attached drawings, wherein:
- Figure 1 is a perspective view, with parts removed for clarity, of a drying conduit realized according to the teachings of the present invention;
- Figure 2 is a larger-scale section view of the conduit of Figure 1, with parts removed for clarity; and
- Figure 3 schematically shows a sterilization unit, with parts removed for clarity, realized according to the teachings of the present invention and comprising the conduit of Figure 1.

### DETAILED DESCRIPTION

With reference to the attached drawings, number 1 indicates as a whole, a hot fluid, preferably hot air, supply conduit configured to dry articles, in the specific case caps 2 adapted to close containers (not shown) such as bottles or the like, adapted to be filled with a pourable product, in particular a pourable food product.

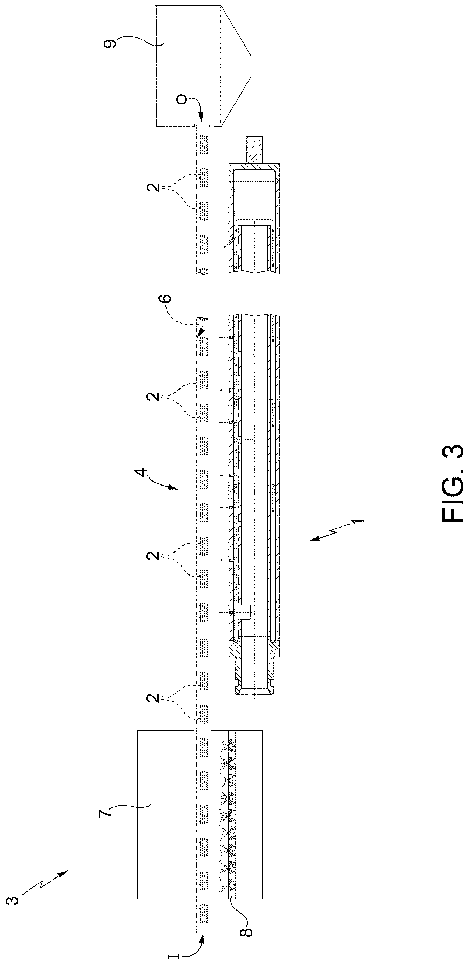

According to the preferred and non-limiting embodiment described herein, the conduit 1 is part of a sterilization unit 3 of the aforementioned caps 2, schematically shown as a whole in Figure 3 and configured to sterilize the caps 2, before applying the same to respective containers.

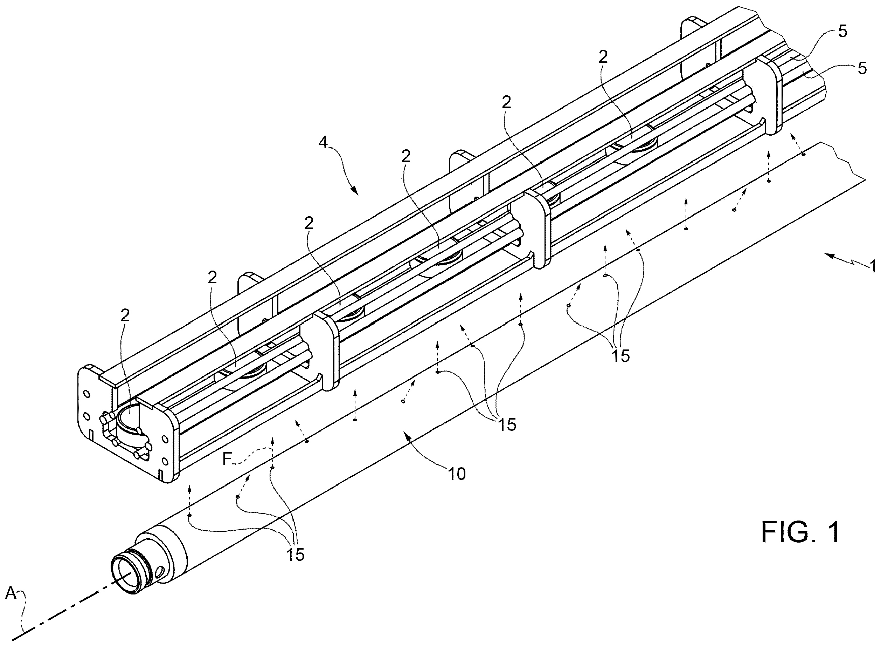

As can be seen in Figure 3, the unit 3 further comprises:
- a conveying structure 4 defined by a plurality of guide bars 5 (Figure 1) arranged so as to delimit a guide channel 6 configured to receive the caps 2 at an input station I and convey the caps towards an output station 0; and
- a treatment chamber 7, operatively interposed between the input station I and the output station 0 and within which the sterilization occurs.

In detail, the caps 2 are conveyed inside the treatment chamber 7 by means of the conveying structure 4, which crosses the treatment chamber 7 itself.

In greater detail the treatment chamber 7 includes an injection pipe 8, preferably positioned below the guide channel 6 defined by the conveying structure 4 and adapted to inject a sterilizing agent (for example heated and vaporized hydrogen peroxide, i.e. VHP) inside the treatment chamber 7 itself.

In this way, a mist of sterilizing agent is formed through which the caps 2 pass during the sterilization process.

The conduit 1 is configured to supply a flow of hot fluid, preferably a flow F of hot air towards the conveying structure 4, so as to touch the caps 2 that slide therein and dry them of any possible residues of sterilizing agent.

To this aim, the conduit 1 is arranged outside of the treatment chamber 7, in particular in a position downstream of the treatment chamber 7 in respect to an advancing direction of the caps from the input station I to the output station 0.

In greater detail, the conduit 1 is positioned below the guide channel 6, so that the flow F that touches the caps 2 from below, as will be explained more clearly in the following.

As can be seen in Figure 3, at the end of the sterilization and drying process, the caps 2 are fed from the unit 3 through the output station 0 to a capping device 9.

In detail, the capping device 9 is arranged downstream of the conduit 1, in particular downstream of the output station 0, and is configured to apply each individual cap 2 to a respective container.

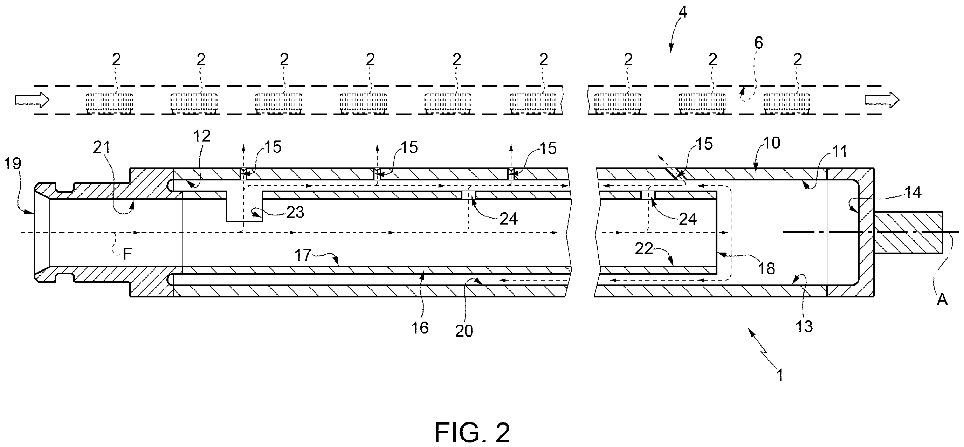

As shown in Figure 2, the conduit 1 comprises a first hollow tubular wall 10 extending along a preferably straight axis A, and internally defining a first flow channel 11 for the hot air.

In detail the first channel 11 has a first axial end portion 12 and a second axial end portion 13 arranged on the axially opposite side with respect to the first portion 12.

In greater detail the first end portion 12 is situated at the end of the conduit 1 closest to the input station I, whereas the second end portion 13 is situated at the end of the conduit 1 closest to the output station 0.

In the example shown, the first tubular wall 10 has a substantially annular-shaped cross-section and comprises a back wall 14, arranged at the second end portion 13 and configured to axially close the first channel 11.

In greater detail, the back wall 14 is coaxial to the axis A and has a substantially circular-shaped cross-section.

According to this preferred and non-limiting embodiment, the first channel 11 is configured to receive the flow F of hot air and to feed it to the caps 2 through a plurality of outlet holes, in particular outlet holes 15 obtained in the tubular wall 10.

In detail, the outlet holes 15 are distributed on the first tubular wall 10 along a longitudinal band substantially parallel to the axis A.

In greater detail, the outlet holes 15 have respective axes extending in a substantially radial direction with respect to the axis A, so that the flow F passes through the outlet holes 15 in a substantially radial direction with respect to the axis A.

In practice, the longitudinal band is arranged below the conveying structure 4 and facing the respective lower portions of the caps 2, when the latter slide within the guide channel 6, thus allowing the flow F flowing out of the outlet holes 15 to touch the caps 2 from below.

According to an important aspect of the present invention, the conduit 1 also comprises a second hollow tubular wall 16, coaxial to the axis A and arranged, at least in part, inside the first tubular wall 10, in particular being surrounded, at least in part, by the first tubular wall 10.

Second tubular wall 16 defines a second flow channel 17 for the hot air, having:
- an inlet opening 19, coaxial to the axis A and configured to receive the hot air from an external hot air source (known per se and not described in detail); and
- a through opening 18 coaxial to the axis A and adapted to be fluidically connected to the inlet opening 19 and to the outlet holes 15.

In detail, the through opening 18 is arranged facing the back wall 14. In greater detail, the through opening 18 is arranged at the second end portion 13, at a distance from the back wall 14 other than zero.

In light of what has been described above, the second channel 17 is configured to convey the hot air from the inlet opening 19 towards the through opening 18 and, therefore, towards the second end portion 13 of the first channel 11, through the through opening 18.

Furthermore, the first tubular wall 10 and the second tubular wall 16 radially delimit between one another, an annular region 20 of the first channel 11. Such annular region 20 is configured to convey hot air from the second end portion 13 to the first end portion 12.

In particular, the flow F of hot air that reaches the second end portion 13, by flowing out of the second channel 17 through the through opening 18, is deflected from the back wall 14 and directed, through the annular region 20, towards the first end portion 12.

As visible in Figure 2, the second channel 17 has an end leading portion 21 and an end trailing portion 22 arranged at respective opposite axial ends of the second channel 17.

In the specific embodiment described herein, the inlet opening 19 is arranged at the leading portion 21, in particular at the end of the second channel 17 closest to the treatment chamber 7; the through opening 18 is arranged at the trailing portion 22, in particular at the end of the second channel 17 closest to the back wall 14.

In practice, the second channel 17 is configured to convey the hot air along a first flow direction, from the inlet opening 19 to the through opening 18, whereas the annular region 20 of the first channel 11 is configured to convey the hot air at least along a second flow direction from the second end portion 13 to the first end portion 12 and, therefore, countercurrent to the first flow direction.

In other words, the configuration of the conduit 1 described above allows a circulation of the flow F in two countercurrent directions.

Furthermore, when passing through the annular region 20, the flow F gradually flows out through the outlet holes 15, distributed along the entire length of the first tubular wall 10, from the second end portion 13 to the first end portion 12.

In this way, only part of the flow F reaches the first end portion 12 and, subsequently, flows out from the particular outlet holes 15 arranged at the end portion 12 itself.

This allows a more efficient heat distribution to be obtained and, therefore, a substantially uniform temperature profile along the entire conduit 1, compared to the case in which the flow F runs in only one direction.

According to a further aspect of the present invention, the second channel 17 has at least a further through opening, in the illustrated case a slit 23 adapted to be fluidically connected to the inlet opening 19 and to the outlet holes 15.

In particular, the slit 23 is configured to allow a spill of hot air from the second channel 17 to the first channel 11, i.e. to allow the passage of part of the flow F from the second channel 17 to the first channel 11, while the flow runs from the inlet opening 19 to the through opening 18.

According to this preferred non-limiting embodiment, the slit 23 is arranged downstream of the inlet opening 19 and upstream of the through opening 18, with respect to the above-mentioned first flow direction of the hot air inside the second channel 17.

In greater detail, the slit 23 is obtained in the second tubular part 26, so that the hot air passes there through, in use, in a substantially radial direction in respect to the axis A.

In other words, part of the flow F is drained from the slit 23, i.e. is fed from the second channel 17 to the first channel 11, before reaching the through opening 18.

Thanks to this configuration of the conduit 1, part of the flow F runs through the annular region 20 according to the above-mentioned first flow direction, i.e. from the slit 23 to the second end portion 13.

This allows to achieve an even more efficient heat distribution and, therefore, a more homogeneous temperature profile along the entire conduit.

As can be seen in Figure 2, the second channel 17 comprises a plurality of further through openings 24, obtained in the tubular wall 16, arranged downstream of the inlet opening 19 and arranged upstream of the through opening 18, in respect to the abovementioned first flow direction, and configured to allow respective spills of hot air from the second channel 17 to the first channel 11.

The presence of further through openings 24 allows to achieve an even more homogeneous heat distribution along the conduit 1, especially in the central area of the conduit 1, i.e. along the annular region 20, until reaching an optimal number of further through openings 24.

The operation of the conduit 1 according to the present invention will be described in the following, with particular reference to Figure 2.

In particular, the flow F of hot air is fed to the second channel 17 through the inlet opening 19 and from the latter, the hot air is conveyed towards the through opening 18 through which it is fed to the second end portion 13 of the first channel 11. During this step, the air transfers part of its heat to the second tubular wall 16.

At this point, due to the shape of the conduit 1, the hot air is deflected by the back wall 14 and directed so as to flow within the annular region 20, towards the first end portion 12 of the first channel 11.

At the same time, part of the flow F is spilled from the second channel 17 to the first channel 11 through the slit 23 and the further through openings 24.

In particular, the spilled part of the flow F runs through the annular region 20 in a direction opposite to the part of the flow F flowing out of the through opening 18 and deflected by the back wall 14.

When passing inside the first channel 11, the air transfers part of its heat to the first tubular wall 10.

Finally, the entire flow F entering into the conduit 1 through the inlet opening 19, flows out of the outlet holes 15.

From an examination of the characteristics of the conduit 1, implemented according to the present invention, the advantages that it allows to obtain are clear.

In particular the presence of a second tubular wall 16, housed coaxially within the first tubular wall 10 and defining a second channel 17 for the flow of hot air, allows to obtain a temperature profile that is substantially uniform along the entire conduit, without the need to increase the temperature of the hot air or the sterilization time of the conduit 1.

This allows the conduit 1 to reach a degree of optimal sterilization without the use of any chemical agent.

Furthermore, the configuration of the conduit 1, described in the present invention allows to obtain a uniform velocity profile of the flow F at the outlet holes 15, thus avoiding reduced intensities of the flow F, especially at the ends of the conduit 1. This is particularly advantageous in the production phase, in particular in the drying step, since the drying degree of the caps 2 remains virtually unchanged along the entire portion of the conveying structure 4 above the conduit 1.

It is clear that variations and modifications can be made to the conduit 1 described and illustrated herein, without departing from the scope of protection defined by the claims.

## Claims

1. A hot fluid supply conduit (1) configured to dry articles (2), comprising a first tubular wall (10) extending along an axis (A) and internally defining a first flow channel (11) for said hot fluid having a first axial end portion (12) and a second axial end portion (13) arranged on axially opposite end of said first channel (11) with respect to said first end portion (12); said first tubular wall (10) comprising a back wall (14) arranged at said second end portion (13) and configured to axially close said first channel (11); said first channel (11) being configured to receive said hot fluid and feed it to said articles (2) through a plurality of outlet openings (15) obtained in said first tubular wall (10);
**characterised in that** it further comprises a second tubular wall (16), arranged at least partially inside said first tubular wall (10) and defining a second flow channel (17) for said hot fluid having a through opening (18) adapted to be fluidically connected to said outlet openings (15) ;
said through opening (18) facing said back wall (14) and being arranged at a distance other than zero from said back wall (14);
said second channel (17) being configured to convey said hot fluid towards said second end portion (13) of said first channel (11), through said through opening (18);
said first tubular wall (10) and said second tubular wall (16) radially delimiting, between one another, an annular region (20) of said first channel (11) configured to convey said hot fluid from said second end portion (13) to said first end portion (12).

2. Conduit according to claim 1, wherein said second channel (17) has at least one inlet opening (19) configured to receive said hot fluid from an external source of said hot fluid and to be fluidically connected to said through opening (18);
said second channel (17) being configured to convey said hot fluid along a first flow direction, from said inlet opening (19) to said through opening (18);
said annular region (20) of said first channel (11) being configured to convey said hot fluid at least in a second flow direction countercurrent to said first flow direction.

3. Conduit according to claim 2, wherein said second channel (17) has at least a further through opening (23, 24) apt to be fluidically connected to said inlet opening (19) and to said outlet openings (15);
said further through opening (23, 24) being configured to allow a spill of said hot fluid from said second channel (17) towards said first channel (11).

4. Conduit according to claim 3, wherein said second channel (17) comprises a leading end portion (21) and a trailing end portion (22) arranged at respective opposite ends of said second channel (17); said inlet opening (19) of said second channel (17) being arranged at said leading portion (21); said through opening (23) being arranged at said trailing portion (22); said further through opening (23) being arranged downstream of said inlet opening (19) and upstream of said through opening (18), with respect to said first flow direction of said hot fluid.

5. Conduit according to claim 4, wherein said further through opening (23) is obtained on said second tubular wall (16); said hot fluid passing through, in use, said further through opening (23) in a substantially radial direction with respect to said axis (A).

6. Conduit according to any one of the preceding claims, wherein said outlet openings (15) are distributed on said first tubular wall (10) along at least a longitudinal band substantially parallel to said axis (A).

7. Conduit according to claim 6, wherein said outlet openings (15) have respective axes extending in a substantially radial direction with respect to said axis (A); said hot fluid passes through said outlet openings (15) in a substantially radial direction with respect to said axis (A).

8. Conduit according to any one of the preceding claims, wherein said second tubular wall (16) is coaxial to said axis (A); said inlet opening (19) and said through opening (18) being coaxial to said axis (A).

9. Conduit according to any one of the preceding claims, wherein said axis (A) is substantially straight.

10. A sterilization unit (3) for articles (2) comprising:
- a conveying structure (4) for said articles (2);
- a treatment chamber (7), through which said conveying structure (4) passes and configured to sterilize, internally thereto, said articles (2); and
- a supply conduit (1) as claimed in any one of the preceding claims;
said conduit (1) being arranged in series and downstream of said treatment chamber (7), with respect to a conveying direction of said articles (2) and, being configured to dry said articles (2) after the sterilization of said articles (2) carried out inside said treatment chamber (7).

11. A system configured to dry caps (2) of containers adapted to be filled with a pourable product, comprising a supply conduit (1) as claimed in any one of the preceding claims.
